# FASCICULE DE BREVET EUROPEEN

(11) **EP 3 419 571 B1**
(45) Date de publication et mention de la délivrance du brevet: **08.04.2020**
(21) Numéro de dépôt: 17712924.4
(22) Date de dépôt: 14.02.2017
(51) Int. Cl.: A61F 5/56

(54) **DISPOSITIF POUR REDUIRE DES NUISANCES SONORES LIEES AU RONFLEMENT**
VORRICHTUNG ZUR REDUZIERUNG VON GERÄUSCHBELÄSTIGUNG IM ZUSAMMENHANG MIT SCHNARCHEN
DEVICE FOR REDUCING THE NOISE NUISANCE LINKED TO SNORING

(30) Priorité: 26.02.2016 BE 201605139
(43) Date de publication de la demande: 02.01.2019
(73) Titulaire: Cavaleri, Antonio, 4420 Saint Nicolas (BE); Ungerman, Charles Warren, 3700 Tongeren (BE); Trovo, Ernesto, 4460 Grace-Hollogne (BE)
(72) Inventeur: Cavaleri, Antonio, 4420 Saint Nicolas (BE); Ungerman, Charles Warren, 3700 Tongeren (BE); Trovo, Ernesto, 4460 Grace-Hollogne (BE)
(74) Mandataire: Gevers Patents
(86) Numéro de dépôt international: PCT/EP2017/053299
(87) Numéro de publication internationale: WO 2017/144311

(56) Documents cités:
- EP-A1- 2 514 470
- WO-A1-2014/032196
- CA-A1- 2 505 364
- US-A1- 2003 121 520

## Description

### Domaine technique

La présente invention concerne un dispositif pour réduire des nuisances sonores liées au ronflement.

### Art antérieur

Le document CA 2 505 364 A1 divulgue un embout buccal pour réduire le ronflement comprenant un corps disposé dans la bouche d'un utilisateur avec un conduit disposé entre les lèvres de l'utilisateur créant un grand passage d'air entre l'intérieur de la bouche et l'environnement extérieur.

Le problème de ce dispositif connu est que le grand passage d'air permanent entre les lèvres de l'utilisateur incite l'utilisateur à une transition d'une respiration nasale à une respiration buccale pendant son sommeil, alors que la respiration nasale est en général préférable à la respiration buccale.

### Résumé de l'invention

Un des buts de l'invention est de fournir un dispositif capable de réduire des nuisances sonores liées au ronflement, qui permette une respiration nasale et une respiration buccale et qui n'incite pas au passage de la respiration nasale à la respiration buccale pendant le sommeil. A cet effet, l'invention propose un dispositif pour réduire des nuisances sonores liées au ronflement et comprenant:
- un écran agencé pour être disposé entre des lèvres et des dents d'un utilisateur et ayant:
   ∘ une première surface agencée pour être en contact avec les lèvres de l'utilisateur, et
   ∘ une deuxième surface agencée pour être en contact avec les dents de l'utilisateur,
   ∘ un conduit traversant la première surface et la deuxième surface et formant une protubérance sur la première surface de l'écran, le conduit étant agencé pour être disposé entre les lèvres de l'utilisateur, le conduit ayant un creux délimité par une surface intérieure du conduit, le creux débouchant sur:
      ▪ une première ouverture agencée pour s'ouvrir vers l'extérieur de la bouche de l'utilisateur, et
      ▪ une deuxième ouverture agencée pour s'ouvrir vers l'intérieur de la bouche de l'utilisateur,
caractérisé en ce que le dispositif comprend en outre un élément d'obstruction couplé à la surface intérieure du conduit et en ce que le dispositif est agencé de façon à ce que l'élément d'obstruction:
- prenne une première position dans laquelle il obstrue au moins partiellement le creux du conduit,
- prenne, sous l'action d'une phase d'expiration buccale de l'utilisateur, une deuxième position dans laquelle il obstrue moins le creux du conduit que dans la première position de façon à permette une sortie d'air d'expiration, et
- prenne, sous l'action d'une phase d'Inspiration buccale de l'utilisateur, une troisième position dans laquelle il obstrue moins le creux du conduit que dans la première position de façon à permettre une entrée d'air d'inspiration, la deuxième position et la troisième position étant situées de part et d'autre de la première position.

L'écran du dispositif selon l'invention crée une protection acoustique capable de réduire l'amplitude des ondes sonores de ronflement créées dans la bouche. Les nuisances sonores liées au ronflement, c'est-à-dire le son de ronflement perçu par une autre personne que le dormeur source du ronflement, sont donc réduites.

En outre, le dispositif selon l'invention permet une respiration nasale car il n'est pas présent au niveau des voies de respiration nasale. Il permet aussi une respiration buccale car l'expiration d'air déplace l'élément d'obstruction dans la deuxième position dans laquelle une sortie d'air d'expiration est possible et l'inspiration d'air déplace l'élément d'obstruction dans la troisième position dans laquelle une entrée d'air d'inspiration est possible.

Lorsque l'utilisateur est en respiration nasale, il n'a pas d'inspiration ou d'expiration. L'élément d'obstruction est dans ce cas dans la première position dans laquelle il obstrue au moins partiellement le conduit. Il n'y a donc pas de grand passage d'air permanent entre les lèvres qui inciterait à une transition de la respiration nasale vers la respiration buccale.

Il est intéressant de constater que le dispositif selon l'invention permet aussi de diminuer le risque d'apnée du sommeil. En effet, des apnées du sommeil peuvent se produire lorsque le nez est bouché et les lèvres sont jointes car il est alors impossible au dormeur de respirer. Chez un dormeur utilisant le dispositif selon l'invention, si le nez se bouche alors que les lèvres sont jointes autour du conduit du dispositif, la respiration buccale est encore possible via le creux du conduit du dispositif.

La deuxième surface est préférentiellement essentiellement parallèle à la première surface.

Dans une réalisation de l'invention, l'élément d'obstruction obstrue partiellement le creux du conduit dans au moins l'une des deuxième position et troisième position.

De façon préférée, l'élément d'obstruction est une languette.

De façon préférée, l'élément d'obstruction est flexible.

Préférentiellement, l'élément d'obstruction comprend un moyen de rappel agencé pour le ramener de la deuxième position vers la première position et de la troisième position vers la première position.

Ce moyen de rappel permet que l'élément d'obstruction retrouve sa position de repos, où il obstrue d'avantage le creux du conduit, lorsque l'utilisateur passe d'une respiration buccale à une respiration nasale.

Dans une réalisation de l'invention, le dispositif comprend un embout percé et amovible, agencé pour être fixé sur la première ouverture du conduit de façon à l'obstruer partiellement.

L'embout étant fabriqué de manière séparée de la fabrication du reste du dispositif, ce dernier est plus facile à fabriquer par moulage par injection. En outre, l'embout peut être changé si l'utilisateur désire avoir plus ou moins d'arrivée d'air.

De manière avantageuse, que l'écran comprend en outre un moyen d'assouplissement agencé pour assouplir au moins partiellement l'écran.

Le moyen d'assouplissement permet de rendre l'écran plus souple, plus flexible, c'est-à-dire plus agréable à porter en bouche.

Préférentiellement, le moyen d'assouplissement est agencé pour assouplir un pourtour de l'écran.

Cela permet de rendre ce pourtour confortable au niveau des gencives. Dans le cadre du présent document, un "pourtour de l'écran" peut être une partie de l'écran située à moins de 1 cm du bord de l'écran, préférentiellement à moins de 2 mm du bord de l'écran.

En outre, le moyen d'assouplissement peut comprendre une rainure sur la première surface de l'écran.

De plus, le moyen d'assouplissement peut comprendre une rainure sur la deuxième surface de l'écran.

Les rainures permettent à l'écran de se plier davantage afin de se conformer à la configuration de l'espace entre les dents ou gencives et les lèvres.

Dans une réalisation de l'invention, l'écran, le conduit et l'élément d'obstruction sont d'un seul tenant.

Avoir ces différentes parties en une seule pièce permet d'éviter des interstices entre ces parties dans lesquels des moisissures pourraient se développer. C'est donc particulièrement hygiénique.

Dans une réalisation de l'invention, l'écran comprend en outre deux ergots en saillie sur la deuxième surface de l'écran.

Ces ergots permettent de maintenir un passage d'air entre l'avant des dents et l'arrière des dents, lorsque les mâchoires se referment.

De manière avantageuse, l'écran, le conduit, l'élément d'obstruction et les ergots sont d'un seul tenant.

Avoir ces différentes parties en une seule pièce permet d'éviter des interstices entre ces parties dans lesquels des moisissures pourraient se développer. C'est donc particulièrement hygiénique.

Dans une réalisation de l'invention, le dispositif comprend au moins un matériau parmi : un polymère, du caoutchouc, du silicone, un polymère à base de caoutchouc, un polymère à base de silicone, du silicone sanitaire, du silicone biocompatible, du caoutchouc biocompatible, un polymère à base de caoutchouc biocompatible, un polymère à base de silicone biocompatible, un polymère prévu pour une injection mécanique, du caoutchouc prévu pour une injection mécanique, du silicone prévu pour une injection mécanique, un polymère à base de caoutchouc prévu pour une injection mécanique, un polymère à base de silicone prévu pour une injection mécanique, du silicone sanitaire prévu pour une injection mécanique, du silicone biocompatible prévu pour une injection mécanique, du caoutchouc biocompatible prévu pour une injection mécanique, un polymère à base de caoutchouc biocompatible prévu pour une injection mécanique et un polymère à base de silicone biocompatible prévu pour une injection mécanique.

Ces matériaux sont flexibles, hygiéniques et compatibles avec l'environnement buccal. En outre utiliser un matériau injectable mécaniquement permet de fabriquer le dispositif, en tout ou en partie, par moulage par injection.

L'invention propose en outre un ensemble comprenant le dispositif selon l'une quelconque des revendications précédentes et un boitier comprenant un emplacement prévu pour le dispositif.

Un tel boitier permet de ranger le dispositif de façon hygiénique.

Dans une réalisation de l'invention, le boitier comprend en outre un support agencé pour recevoir au moins un embout du dispositif.

Un tel support permet de ranger l'ensemble du dispositif dans un même boitier, en évitant des espaces creux entre l'embout et le conduit dans lesquels des bactéries pourraient se développer.

Dans une réalisation de l'invention, le boitier comprend en outre à l'emplacement prévu pour le dispositif, un support pour le dispositif qui est plein et agencé pour soutenir la deuxième surface du dispositif.

Un tel support permet de permet de minimiser la quantité de liquide de nettoyage nécessaire pour baigner le dispositif.

Une possible méthode pour diminuer des ronflements d'un utilisateur en utilisant le dispositif selon l'invention comprend les étapes de:
- fournir le dispositif selon l'une quelconque des revendications précédentes, et
- placer le dispositif de façon à ce que la première surface de l'écran soit en contact avec les lèvres de l'utilisateur, la deuxième surface soit en contact avec les dents de l'utilisateur et le conduit soit entre les lèvres de l'utilisateur.

### Brève description des figures

D'autres caractéristiques et avantages de l'invention apparaîtront à la lecture de la description détaillée qui suit pour la compréhension de laquelle on se reportera aux figures annexées parmi lesquelles :
- la figure 1 illustre une vue avant d'un dispositif selon une réalisation de l'invention,
- la figure 2 illustre trois embouts possibles selon une réalisation de l'invention,
- la figure 3 illustre une vue arrière du dispositif selon une réalisation de l'invention
- la figure 4 illustre une vue du dessus du dispositif sans l'embout, avec notamment des ergots et un moyen de fixation selon une réalisation de l'invention,
- la figure 5 illustre trois positions possibles d'un élément d'obstruction dans une réalisation de l'invention,
- la figure 6 illustre un boitier destiné à recevoir le dispositif dans une réalisation de l'invention, et
- la figure 7 illustre une vue en coupe latérale du boitier dans une autre réalisation de l'invention.

### Modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants.

Dans le contexte du présent document, les termes « premier » et « deuxième » servent uniquement à différencier les différents éléments et n'impliquent pas d'ordre entre ces éléments.

Sur les figures, les éléments identiques ou analogues peuvent porter les mêmes références.

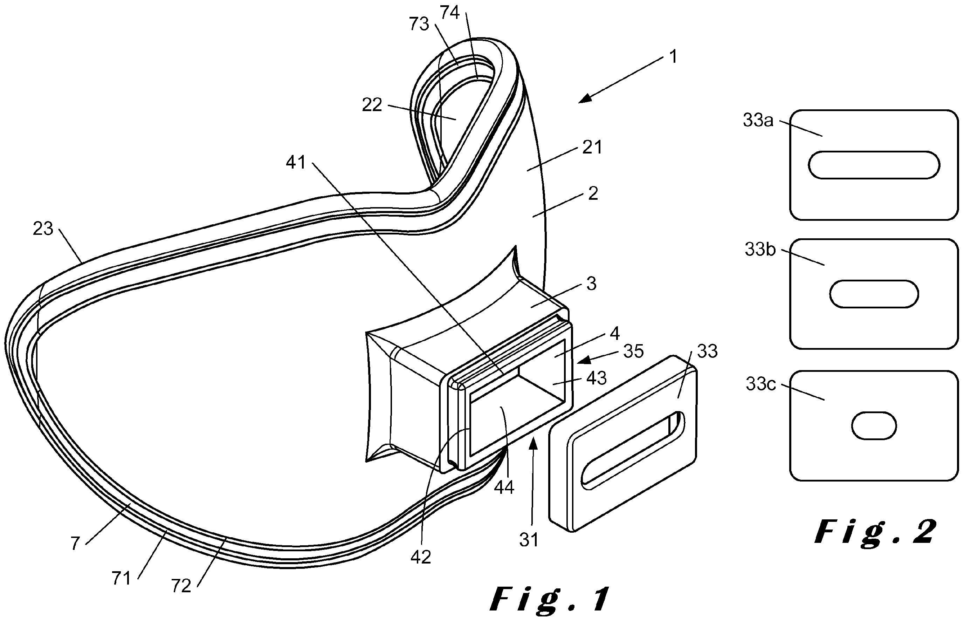

La figure 1 illustre une vue avant d'un dispositif 1 selon une réalisation de l'invention. Le dispositif 1 est destiné à éviter ou diminuer les ronflements et les apnées du sommeil. Le dispositif 1 est destiné à être placé en partie dans la bouche d'une personne ou d'un patient, de manière amovible. Le dispositif 1 est fait d'un matériau souple et ne présentant pas de risque de fuite dans l'environnement buccal. Par exemple, le dispositif 1 peut être fait d'un polymère compatible avec l'environnement buccal comme un polymère, du caoutchouc, du silicone, un polymère à base de caoutchouc, un polymère à base de silicone, du silicone sanitaire, du silicone biocompatible, du caoutchouc biocompatible, un polymère à base de caoutchouc biocompatible, un polymère à base de silicone biocompatible, un polymère prévu pour une injection mécanique, du caoutchouc prévu pour une injection mécanique, du silicone prévu pour une injection mécanique, un polymère à base de caoutchouc prévu pour une injection mécanique, un polymère à base de silicone prévu pour une injection mécanique, du silicone sanitaire prévu pour une injection mécanique, du silicone biocompatible prévu pour une injection mécanique, du caoutchouc biocompatible prévu pour une injection mécanique, un polymère à base de caoutchouc biocompatible prévu pour une injection mécanique et un polymère à base de silicone biocompatible prévu pour une injection mécanique.

Le dispositif 1 comprend un écran 2, destiné à être placé entre les dents et les lèvres d'un utilisateur et ayant une taille et une forme adéquate pour cela. En particulier, l'écran 2 une première surface 21 agencée pour être en contact avec les lèvres de l'utilisateur qui est convexe et une deuxième surface 22 agencée pour être en contact avec les dents de l'utilisateur qui est concave. L'écran 2 peut par exemple avoir une hauteur entre 3 et 7 cm et une largeur entre 4 et 8 cm. L'écran 2 peut par exemple avoir une profondeur entre 1 et 4 mm.

Dans le présent document, le terme "hauteur" correspond à une dimension verticale lorsque l'écran 2 du dispositif 1 est dans la bouche d'un utilisateur se tenant debout. Les expressions "vers le haut" et "vers le bas" se réfèrent aussi à une telle situation.

Dans le présent document, le terme "largeur" correspond à une dimension gauche-droite pour un utilisateur lorsque l'écran 2 du dispositif 1 est dans sa bouche. Les expressions "vers la droite" et "vers la gauche" se réfèrent aussi à une telle situation.

Dans le présent document, le terme "profondeur" correspond à une dimension devant-derrière pour un utilisateur lorsque l'écran 2 du dispositif 1 est dans sa bouche.

Le dispositif 1 comprend également un conduit 3 traversant la première surface 21 et la deuxième surface 22 et formant une protubérance sur la première surface 21 de l'écran. Le conduit 3 est agencé pour être disposé entre les lèvres de l'utilisateur. Le conduit 3 peut avoir une section rectangulaire ou de toute autre forme. Le conduit 3 a un creux 35 délimité par une surface intérieure 4 du conduit 3. Ce creux 35 débouche d'une part sur une première ouverture 31 agencée pour s'ouvrir vers l'extérieur de la bouche de l'utilisateur, et, d'autre part, sur une deuxième ouverture 32 agencée pour s'ouvrir vers l'intérieur de la bouche de l'utilisateur. Un passage d'air entre la bouche de l'utilisateur et l'extérieur est donc possible via le creux 35. Le creux 3 peut par exemple avoir une hauteur entre 6 et 10 mm et une largeur entre 10 et 18 mm.

Si la section du creux 35 est rectangulaire comme illustré à la figure 1, la surface intérieure 4 du conduit 3 a un premier côté 41, situé vers le haut, un deuxième côté 42, situé vers la droite, un troisième côté 43, situé vers la gauche, et un quatrième côté 44, situé vers le bas.

Le conduit 3 est préférentiellement d'un même matériau et d'un seul tenant avec l'écran 2.

Le dispositif 1 peut comprendre également un moyen d'assouplissement 7 agencé pour assouplir au moins partiellement l'écran 2. Le moyen d'assouplissement 7 permet de rendre l'écran 2 plus souple, plus flexible, c'est-à-dire plus agréable à porter en bouche.

En particulier, le moyen d'assouplissement 7 peut être agencé pour assouplir un pourtour 23 de l'écran 2 de façon à ce rendre ce pourtour 23 confortable au niveau des gencives.

Le moyen d'assouplissement 7 peut comprendre une ou plusieurs rainures, par exemple, le dispositif 1 illustré à la figure 1 comporte deux rainures 71, 72 sur la première surface 21 de l'écran 2 et deux rainures 73, 74 sur la deuxième surface 22 de l'écran 2. Les rainures 71-74 suivent, avec un certain décalage, le bord de l'écran 2. Par exemple, les rainures 71 et 73 peuvent être situées à une distance comprise entre 2 et 3 mm du bord de l'écran. Par exemple, les rainures 72 et 74 peuvent être situées à une distance comprise entre 4 et 6 mm du bord de l'écran.

La première rainure 71 est opposée à la troisième rainure 73, alors que la deuxième rainure 72 est opposée à la quatrième rainure 74. Les rainures 71-74 permettent au pourtour 23 de l'écran 2 de se plier davantage afin de se conformer à la configuration de l'espace entre les gencives et les lèvres.

En outre, si l'écran 2 est trop grand pour l'utilisateur, celui-ci peut utiliser un des rainures 71-74 comme guide afin de découper l'écran 2 à la dimension adéquate.

Le dispositif 1 peut comprendre également un embout 33 percé d'un trou et amovible agencé pour être fixé sur la première ouverture 31 du conduit 3 de façon à l'obstruer partiellement. La figure 2 illustre trois embouts possibles, 33a, 33b, 33c, ayant chacun des trous de sections différentes. Il est possible, tout en restant dans le cadre de l'invention, que l'embout 33 comporte plusieurs trous. En remplaçant un premier embout 33a par un deuxième embout 33b ou un troisième embout 33c ayant des trous de sections différentes, l'utilisateur peut adapter le passage d'air à sa situation, pour avoir plus ou moins d'air. La première ouverture 31 du creux 35 du conduit 3 comporte préférentiellement un moyen de fixation 34 de l'embout 33, par exemple une partie femelle dans laquelle peut venir s'engager une partie mâle présente sur l'embout 33. Le moyen de fixation 34 est prévu pour que la fixation et la libération de l'embout 33 puissent se faire manuellement. L'embout 33 est préférentiellement fait dans le ou les même(s) matériau(x) que l'écran 2 et le conduit 3.

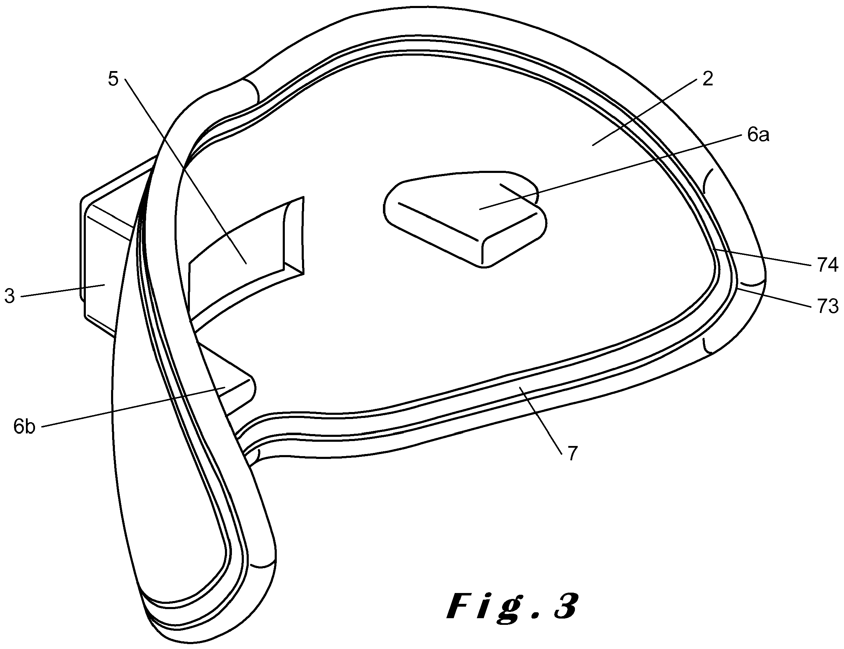

La figure 3 illustre une vue arrière du dispositif 1 selon une réalisation de l'invention.

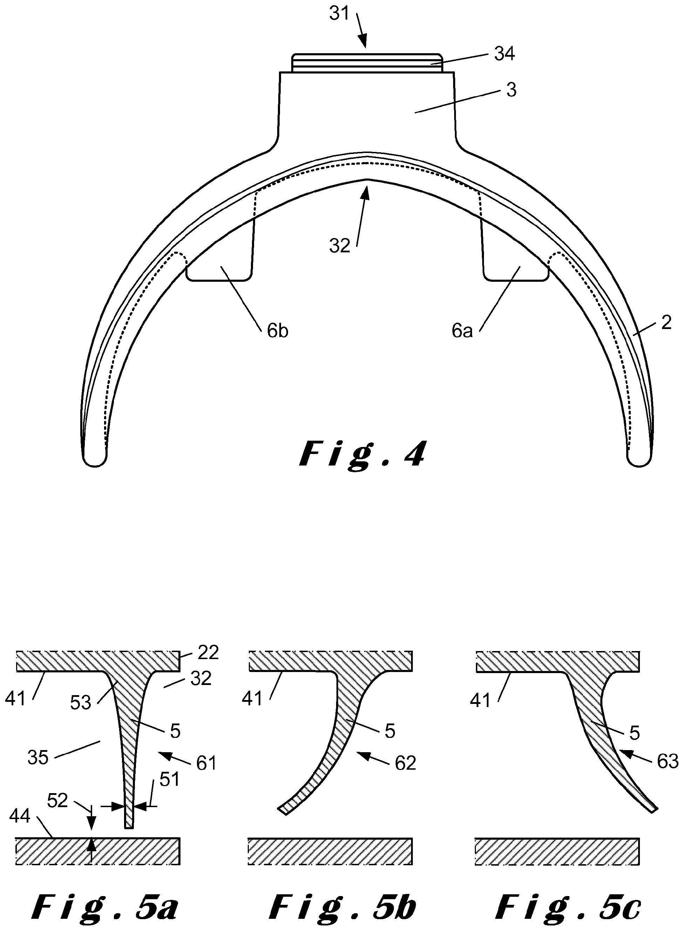

Le dispositif 1 peut comprendre des ergots 6a, 6b, en nombre pair de façon à être situés à gauche et à droite de la bouche, et destinés à maintenir un passage d'air entre l'avant des dents et l'arrière des dents, lorsque les mâchoires se referment. Les ergots 6a, 6b sont en saillie sur la deuxième surface 22 de l'écran 2. Les ergots 6a, 6b sont situés de manière à se placer au niveau des canines. Les ergots 6a, 6b peuvent avoir une forme incurvée vers la mâchoire inférieure et façon à s'accrocher à celle-ci ou peuvent être essentiellement plans. Les ergots 6a, 6b ont préférentiellement une largeur, prise parallèlement à la deuxième surface 22, comprise entre 6 et 10 mm, une profondeur, prise perpendiculairement à la deuxième surface 22, comprise entre 6 et 10 mm, et une hauteur comprise entre 2 et 4 mm. Les ergots 6a, 6b sont préférentiellement d'un même matériau et d'un seul tenant avec l'écran 2. La figure 4 illustre une vue du dessus du dispositif 1 sans l'embout 33, illustrant notamment les ergots 6a, 6b et le moyen de fixation 34.

Le dispositif 1 comprend un élément d'obstruction 5 couplé mécaniquement, par exemple fixé, à la surface intérieure 4 du conduit 3. L'élément d'obstruction 5 est préférentiellement fixé sur le premier côté 41 de la surface intérieure 4 du conduit 3. L'élément d'obstruction 5 est préférentiellement fixé plus près de la deuxième ouverture 32 du creux 35 que de la première ouverture 31 du creux 35. L'élément d'obstruction 5 est préférentiellement d'un seul tenant avec le conduit 3. L'élément d'obstruction 5 est préférentiellement une languette. L'élément d'obstruction 5 est préférentiellement flexible.

La figure 5 illustre trois positions possibles de l'élément d'obstruction 5. La figure 5a illustre une première position 61, dans laquelle l'élément d'obstruction 5 obstrue en grande partie le creux 35 du conduit 3, empêchant ainsi le passage d'air entre l'extérieur et l'intérieur de la bouche par le conduit 3. La première position 61 correspond à une situation "au repos" de l'élément d'obstruction 5 dans laquelle l'élément d'obstruction 5 n'est sujet à aucune force en dehors de la gravité.

La figure 5b illustre une deuxième position 62, dans laquelle l'élément d'obstruction 5 obstrue beaucoup moins le creux 35 du conduit 3 que dans la première position 61, permettant ainsi un passage d'air pour l'expiration entre l'intérieur et l'extérieur de la bouche par le conduit 3, c'est-à-dire une sortie d'air d'expiration. L'élément d'obstruction 5 se met dans la deuxième position 62 sous l'effet de l'expiration de l'utilisateur.

La figure 5c illustre une troisième position 63, dans laquelle l'élément d'obstruction 5 obstrue beaucoup moins le creux 35 du conduit 3 que dans la première position 61, permettant ainsi une passage d'air pour l'inspiration entre l'extérieur et l'intérieur de la bouche par le conduit 3, c'est-à-dire une entrée d'air d'inspiration. L'élément d'obstruction 5 se met dans la troisième position 63 sous l'effet de l'inspiration de l'utilisateur.

Dans la réalisation de l'invention illustrée à la figure 5, les deuxième position 62 et troisième position 63 sont de part et d'autre de la première position 61, la première position 61 étant intermédiaire entre la deuxième position 62 et la troisième position 63. Le mouvement de l'élément d'obstruction 5 peut alors suivre le rythme de la respiration buccale du dormeur.

Dans la première position 61, l'élément d'obstruction 5 obstrue préférentiellement plus de 80% de la section du creux 35, plus préférentiellement plus de 90%, encore plus préférentiellement plus de 95%.

Dans la deuxième position 62, l'élément d'obstruction 5 obstrue préférentiellement moins de 50% de la section du creux 35, plus préférentiellement moins de 35%, encore plus préférentiellement moins de 15%. Dans une réalisation de l'invention, suivant la pression due à l'expiration, l'élément d'obstruction 5 obstrue entre 15 et 35% de la section du creux 35.

Dans la troisième position 63, l'élément d'obstruction 5 obstrue préférentiellement moins de 50% de la section du creux 35, plus préférentiellement moins de 35%, encore plus préférentiellement moins de 10%.

L'élément d'obstruction 5 permet donc le passage de l'air à travers le creux 35 du conduit 3 lorsqu'il subit une différence de pression, dans un sens ou dans l'autre sens, au-delà d'un certain seuil. L'élément d'obstruction 5 suit le mouvement de l'air lors de l'inspiration et l'expiration.

L'élément d'obstruction 5 a préférentiellement une épaisseur 51 de 0.2 à 0.4 mm à son extrémité non-fixée. L'élément d'obstruction 5 comprend préférentiellement un moyen de rappel 53 agencé pour ramener l'élément d'obstruction de la deuxième position 62 vers la première position 61 et de la troisième position 63 vers la première position 61. Le moyen de rappel 53 comprend par exemple un évasement de l'élément d'obstruction 5 à proximité de sa fixation à la surface intérieure 4 du conduit 3, cet évasement favorisant une tendance de l'élément d'obstruction 5 à reprendre sa forme d'origine, c'est-à-dire à revenir à la première position 61.

Le côté non-fixé de l'élément d'obstruction 5 est préférentiellement à une distance 52 entre 1 et 2 mm de la surface intérieure 4 du conduit 3, c'est-à-dire du quatrième côté 44. Le côté droit de l'élément d'obstruction 5 est préférentiellement à une distance entre 1 et 2 mm du deuxième côté 42. Le côté gauche de l'élément d'obstruction 5 est préférentiellement à une distance entre 1 et 2 mm du troisième côté 43.

L'écran 2, le conduit 3, les ergots 6 et l'élément d'obstruction 5 sont préférentiellement fabriqués d'un seul tenant par moulage par injection.

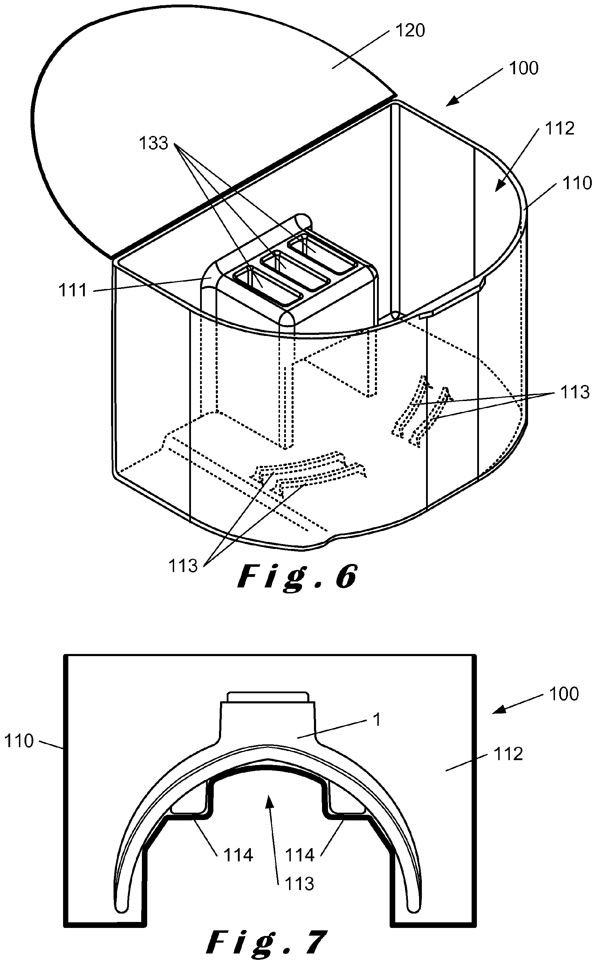

La figure 6 illustre un boitier 100 comprenant un récipient 110 destiné à recevoir le dispositif 1 et un couvercle 120, dans une réalisation de l'invention. Le récipient 110 comporte un emplacement 112 prévu pour le dispositif 1. L'emplacement 112 prévu pour le dispositif 1 peut comprendre des éléments de blocage 113, par exemple sous la forme de saillies dans le fond du récipient, permettant de bloquer le dispositif à l'emplacement 112 prévu pour lui.

Le récipient 110 est préférentiellement capable de contenir un liquide de nettoyage dans lequel le dispositif 1 peut rester plongé. Le récipient 110 peut comporter un support 111 agencé pour recevoir un ou plusieurs embouts 33, par exemple dans trois cavités 133 prévues pour recevoir trois embouts. Le support 111 pour embouts peut être placé de façon à faire face à la deuxième surface 22 de l'écran 2, au moins partiellement dans la cavité formée par l'écran 2, quand celui-ci est dans le boitier 100, de façon minimise la taille du boitier 100.

La figure 7 illustre une vue en coupe latérale du boitier 100 dans une autre réalisation de l'invention. Le fond du récipient 110 comprend un support 113 pour le dispositif 1 qui permet de minimiser la quantité de liquide de nettoyage. Le support 113 pour le dispositif 1 est d'une forme adaptée pour recevoir la deuxième surface 22 de l'écran 2, et en particulier comporte des encoches 114 prévues pour recevoir les ergots 6a, 6b. Ce support 113 peut être faire partie du fond du récipient si la forme de ce fond est adaptée pour recevoir le dispositif 2.

En d'autres termes, l'invention se rapporte à un dispositif 1 pour éviter ou diminuer les ronflements et les apnées du sommeil. Le dispositif 1 comporte un écran 2 agencé pour être disposé entre les lèvres et les dents d'un utilisateur et un conduit 3 formant une protubérance sur une surface 21 convexe de l'écran. Un élément d'obstruction 5 flexible est fixé dans le creux 5 du conduit 3, l'élément d'obstruction 5 étant agencé pour obstruer le passage d'air dans le conduit 3 en cas de respiration nasale de l'utilisateur et libérer le passage d'air dans le conduit 3 en cas de respiration buccale de l'utilisateur. Le dispositif 1 permet en outre de prévenir les risques d'apnées du sommeil.

La présente invention a été décrite en relation avec des modes de réalisations spécifiques, qui ont une valeur purement illustrative et ne doivent pas être considérés comme limitatifs. D'une manière générale, la présente invention n'est pas limitée aux exemples illustrés et/ou décrits ci-dessus. L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments. Les numéros de référence dans les revendications ne limitent pas leur portée.

## Revendications

1. Dispositif (1) pour réduire des nuisances sonores liées au ronflement et comprenant:
• un écran (2) agencé pour être disposé entre des lèvres et des dents d'un utilisateur et ayant:
∘ une première surface (21) agencée pour être en contact avec les lèvres de l'utilisateur, et
∘ une deuxième surface (22) agencée pour être en contact avec les dents de l'utilisateur,
∘ un conduit (3) traversant la première surface (21) et la deuxième surface (22) et formant une protubérance sur la première surface (21) de l'écran (2), le conduit (3) étant agencé pour être disposé entre les lèvres de l'utilisateur, le conduit (3) ayant un creux (35) délimité par une surface intérieure (4) du conduit (3), le creux (35) débouchant sur:
▪ une première ouverture (31) agencée pour s'ouvrir vers l'extérieur de la bouche de l'utilisateur, et
▪ une deuxième ouverture (32) agencée pour s'ouvrir vers l'intérieur de la bouche de l'utilisateur,
**caractérisé en ce que** le dispositif (1) comprend en outre un élément d'obstruction (5) couplé à la surface intérieure (4) du conduit (3) et **en ce que** le dispositif (1) est agencé de façon à ce que l'élément d'obstruction (5):
• prenne une première position (61) dans laquelle il obstrue au moins partiellement le creux (35) du conduit (3),
• prenne, sous l'action d'une phase d'expiration buccale de l'utilisateur, une deuxième position (62) dans laquelle il obstrue moins le creux (35) du conduit (3) que dans la première position (61) de façon à permette une sortie d'air d'expiration, et
• prenne, sous l'action d'une phase d'Inspiration buccale de l'utilisateur, une troisième position (63) dans laquelle il obstrue moins le creux (35) du conduit (3) que dans la première position (61) de façon à permettre une entrée d'air d'inspiration, la deuxième position (62) et la troisième position (63) étant situées de part et d'autre de la première position (61).

2. Dispositif (1) selon la revendication 1, **caractérisé en ce que** l'élément d'obstruction (5) obstrue partiellement le creux (35) du conduit (3) dans au moins l'une des deuxième position (62) et troisième position (63).

3. Dispositif (1) selon l'une quelconque des revendications précédentes, **caractérisé en ce que** l'élément d'obstruction (5) est une languette.

4. Dispositif (1) selon l'une quelconque des revendications précédentes, **caractérisé en ce que** l'élément d'obstruction (5) est flexible.

5. Dispositif (1) selon l'une quelconque des revendications précédentes, **caractérisé en ce que** l'élément d'obstruction (5) comprend un moyen de rappel (53) agencé pour le ramener de la deuxième position (62) vers la première position (61) et de la troisième position (63) vers la première position (61).

6. Dispositif (1) selon l'une quelconque des revendications précédentes, **caractérisé en ce qu'**il comprend en outre un embout (33) percé et amovible, agencé pour être fixé sur la première ouverture (31) du conduit (3) de façon à l'obstruer partiellement.

7. Dispositif (1) selon l'une quelconque des revendications précédentes, **caractérisé en ce que** l'écran (2) comprend en outre un moyen d'assouplissement (7) agencé pour assouplir au moins partiellement l'écran (2).

8. Dispositif (1) selon la revendication 7, **caractérisé en ce que** le moyen d'assouplissement (7) est agencé pour assouplir un pourtour (23) de l'écran (2).

9. Dispositif (1) selon la revendication 7 ou 8, **caractérisé en ce que** le moyen d'assouplissement (7) comprend une rainure (71) sur la première surface (21) de l'écran (2) et/ou une rainure (73) sur la deuxième surface (22) de l'écran (2).

10. Dispositif (1) selon l'une quelconque des revendications précédentes, **caractérisé en ce que** l'écran (2), le conduit (3) et l'élément d'obstruction (5) sont d'un seul tenant.

11. Dispositif (1) selon l'une quelconque des revendications précédentes, **caractérisé en ce que** l'écran (2) comprend en outre deux ergots (6a, 6b) en saillie sur la deuxième surface (22) de l'écran (2).

12. Dispositif (1) selon la revendication 11, **caractérisé en ce que** l'écran (2), le conduit (3), l'élément d'obstruction (5) et les ergots (6a, 6b) sont d'un seul tenant.

13. Ensemble comprenant le dispositif (1) selon l'une quelconque des revendications précédentes et un boitier (100) comprenant un emplacement (112) prévu pour le dispositif (1).

14. Ensemble selon la revendication 13, **caractérisé en ce que** le boitier (100) comprend en outre un support (111) agencé pour recevoir au moins un embout (33) du dispositif (1).

## Patentansprüche

1. Vorrichtung (1) zum Reduzieren der mit dem Schnarchen verbundenen Lärmbelästigungen und umfassend:
• eine Abschirmung (2), die eingerichtet ist, um zwischen den Lippen und Zähnen eines Benutzers angeordnet zu werden, und aufweist:
∘ eine erste Oberfläche (21), die eingerichtet ist, mit den Lippen des Benutzers in Kontakt zu sein, und
∘ eine zweite Oberfläche (22), die eingerichtet ist, mit den Zähnen des Benutzers in Kontakt zu sein,
∘ eine Leitung (3), die durch die erste Oberfläche (21) und die zweite Oberfläche (22) verläuft und einen Vorsprung auf der ersten Oberfläche (21) der Abschirmung (2) bildet, wobei die Leitung (3) eingerichtet ist, zwischen den Lippen des Benutzers angeordnet zu werden, wobei die Leitung (3) einen Hohlraum (35) aufweist, der durch eine innere Oberfläche (4) der Leitung (3) begrenzt ist, wobei der Hohlraum (35) mündet in:
▪ einer ersten Öffnung (31), die eingerichtet ist, sich zu der Außenseite des Mundes des Benutzers hin zu öffnen, und
▪ einer zweiten Öffnung (32), die eingerichtet ist, sich zu der Innenseite des Mundes des Benutzers hin zu öffnen,
**dadurch gekennzeichnet, dass** die Vorrichtung (1) ferner ein Blockierelement (5) umfasst, das mit der inneren Oberfläche (4) der Leitung (3) gekoppelt ist, und dass die Vorrichtung (1) eingerichtet ist, so dass das Blockierelement (5):
• eine erste Position (61) einnimmt, in der es den Hohlraum (35) der Leitung (3) zumindest teilweise blockiert,
• unter der Wirkung einer Phase des oralen Ausatmens des Benutzers eine zweite Position (62) einnimmt, in der es den Hohlraum (35) der Leitung (3) weniger als in der ersten Position (61) blockiert, um ein Ausatmen von Luft zu ermöglichen und
• unter der Wirkung einer Phase des oralen Einatmens des Benutzers, eine dritte Position (63) einnimmt, in der es den Hohlraum (35) der Leitung (3) weniger als in der ersten Position (61) blockiert, um den Eintritt von Einatmungsluft zu ermöglichen, wobei sich die zweite Position (62) und die dritte Position (63) auf beiden Seiten der ersten Position (61) befinden.

2. Vorrichtung (1) nach Anspruch 1, **dadurch gekennzeichnet, dass** das Blockierelement (5) den Hohlraum (35) der Leitung (3) in mindestens einer der zweiten Position (62) und dritten Position (63) teilweise blockiert.

3. Vorrichtung (1) nach einem der vorhergehenden Ansprüche, **dadurch gekennzeichnet, dass** das Blockierelement (5) eine Zunge ist.

4. Vorrichtung (1) nach einem der vorhergehenden Ansprüche, **dadurch gekennzeichnet, dass** das Blockierelement (5) flexibel ist.

5. Vorrichtung (1) nach einem der vorhergehenden Ansprüche, **dadurch gekennzeichnet, dass** das Blockierelement (5) ein Rückstellmittel (53) umfasst, das eingerichtet ist, um es von der zweiten Position (62) zu der ersten Position (61) und von der dritten Position (63) zu der ersten Position (61) zurückzubringen.

6. Vorrichtung (1) nach einem der vorhergehenden Ansprüche, **dadurch gekennzeichnet, dass** sie ferner einen durchbohrten und entfernbaren Stutzen (33) umfasst, der eingerichtet ist, um an der ersten Öffnung (31) der Leitung (3) befestigt zu sein, um sie teilweise zu blockieren.

7. Vorrichtung (1) nach einem der vorhergehenden Ansprüche, **dadurch gekennzeichnet, dass** die Abschirmung (2) ferner ein Geschmeidigmachungsmittel (7) umfasst, das eingerichtet ist, um die Abschirmung (2) zumindest teilweise geschmeidig zu machen.

8. Vorrichtung (1) nach Anspruch 7, **dadurch gekennzeichnet, dass** das Geschmeidigmachungsmittel (7) eingerichtet ist, um einen Umfang (23) der Abschirmung (2) geschmeidig zu machen.

9. Vorrichtung (1) nach Anspruch 7 oder 8, **dadurch gekennzeichnet, dass** das Geschmeidigmachungsmittel (7) eine Nut (71) auf der ersten Oberfläche (21) der Abschirmung (2) und / oder eine Nut (73) auf der zweiten Oberfläche (22) der Abschirmung (2) umfasst.

10. Vorrichtung (1) nach einem der vorhergehenden Ansprüche, **dadurch gekennzeichnet, dass** die Abschirmung (2), die Leitung (3) und das Blockierelement (5) aus einem Stück sind.

11. Vorrichtung (1) nach einem der vorhergehenden Ansprüche, **dadurch gekennzeichnet, dass** die Abschirmung (2) ferner zwei Stollen (6a, 6b) umfasst, die über die zweite Oberfläche (22) der Abschirmung (2) vorstehen.

12. Vorrichtung (1) nach Anspruch 11, **dadurch gekennzeichnet, dass** die Abschirmung (2), die Leitung (3), das Blockierelement (5) und die Nocken (6a, 6b) aus einem Stück sind.

13. Anordnung, umfassend die Vorrichtung (1) gemäß einem der vorhergehenden Ansprüche und ein Gehäuse (100), das eine Einbaustelle (112) umfasst, die für die Vorrichtung (1) vorgesehen ist.

14. Anordnung nach Anspruch 13, **dadurch gekennzeichnet, dass** das Gehäuse (100) ferner einen Träger (111) umfasst, der so eingerichtet ist, dass er mindestens einen Stutzen (33) der Vorrichtung (1) aufnimmt.

## Claims

1. Device (1) for reducing noise pollution linked to snoring and comprising:
• a screen (2) arranged to be disposed between the lips and the teeth of a user and having:
∘ a first surface (21) arranged to be in contact with the lips of the user, and
∘ a second surface (22) arranged to be in contact with the teeth of the user,
∘ a duct (3) crossing the first surface (21) and the second surface (22) and forming a protrusion over the first surface (21) of the screen (2), the duct (3) being arranged to be disposed between the lips of the user, the duct (3) having a cavity (35) delimited by an inner surface (4) of the duct (3), the cavity (35) leading to:
▪ a first opening (31) arranged to be opened towards the outside of the mouth of the user, and
▪ a second opening (32) arranged to be opened towards the inside of the mouth of the user,
**characterised in that** the device (1) further comprises an obstruction element (5) coupled with the inner surface (4) of the duct (3) and **in that** the device (1) is arranged such that the obstruction element (5):
• takes a first position (61) wherein it obstructs at least partially the cavity (35) of the duct (3),
• takes, under the action of a phase of the user breathing out through the mouth, a second position (62) wherein it obstructs less the cavity (35) of the duct (3) than in the first position (61), to allow for an expiration output of air, and
• takes, under the action of a phase of the user breathing in through the mouth, a third position (63) wherein it obstructs less the cavity (35) of the duct (3) than in the first position (61), so as to allow for an inspiration input of air, the second position (62) and the third position (63) being situated on either side of the first position (61).

2. Device (1) according to claim 1, **characterised in that** the obstruction element (5) partially obstructs the cavity (35) of the duct (3) in at least one of the second (62) and third (63) positions.

3. Device (1) according to any one of the preceding claims, **characterised in that** the obstruction element (5) is a flap.

4. Device (1) according to any one of the preceding claims, **characterised in that** the obstruction element (5) is flexible.

5. Device (1) according to any one of the preceding claims, **characterised in that** the obstruction element (5) comprises a return means (53) arranged to return it from the second position (62) to the first position (61) and from the third position (63) to the first position (61).

6. Device (1) according to any one of the preceding claims, **characterised in that** it further comprises a bored and removable tip (33), arranged to be attached on the first opening (31) of the duct (3) so as to partially obstruct it.

7. Device (1) according to any one of the preceding claims, **characterised in that** the screen (2) further comprises a softening means (7) arranged to soften the screen (2), at least partially.

8. Device (1) according to claim 7, **characterised in that** the softening means (7) is arranged to soften a perimeter (23) of the screen (2).

9. Device (1) according to claim 7 or 8, **characterised in that** the softening means (7) comprises a groove (71) on the first surface (21) of the screen (2) and/or a groove (73) on the second surface (22) of the screen (2).

10. Device (1) according to any one of the preceding claims, **characterised in that** the screen (2), the duct (3) and the obstruction element (5) are integral.

11. Device (1) according to any one of the preceding claims, **characterised in that** the screen (2) further comprises two lugs (6a, 6b) protruding over the second surface (22) of the screen (2).

12. Device (1) according to claim 11, **characterised in that** the screen (2), the duct (3), the obstruction element (5) and the lugs (6a, 6b) are integral.

13. Unit comprising the device (1) according to any one of the preceding claims and a casing (100) comprising a placement (112) provided for the device (1).

14. Unit according to claim 13, **characterised in that** the casing (100) further comprises a support (111) arranged to receive at least one tip (33) of the device (1).
